# FASCICULE DE BREVET EUROPEEN

(11) **EP 1 137 615 B1**
(45) Date de publication et mention de la délivrance du brevet: **22.02.2006**
(21) Numéro de dépôt: 99958253.9
(22) Date de dépôt: 07.12.1999
(51) Int. Cl.: C07B 31/00, C07C 51/377, C07C 53/18

(54) **PROCEDE D'HYDROGENODESHALOGENATION SELECTIVE**
VERFAHREN ZUR SELEKTIVEN HYDRODEHALOGENIERUNG
SELECTIVE HYDRODEHALOGENATION METHOD

(30) Priorité: 11.12.1998 FR 9815684
(43) Date de publication de la demande: 04.10.2001
(73) Titulaire: RHODIA CHIMIE, 92512 Boulogne Billancourt Cedex (FR)
(72) Inventeur: JACQUOT, Roland, F-69340 Francheville (FR); CORDIER, Georges, F-69340 Francheville (FR)
(74) Mandataire: Esson, Jean-Pierre
(86) Numéro de dépôt international: PCT/FR1999/003031
(87) Numéro de publication internationale: WO 2000/035834

(56) Documents cités:
- EP-A- 0 657 413
- EP-A- 0 726 244
- GB-A- 1 364 495
- US-A- 4 873 381
- MIYASHITA O ET AL: "Studies on fluorinated pyrimidines. IV. Stereochemistry of 6-alkoxy-5-fluoro- 5,6-dihydrouracils and 5-alkoxycarbonyl- 5-fluoro-6-substituted-5,6-dihydrouracils" CHEMICAL AND PHARMACEUTICAL BULLETIN, vol. 30, no. 7, juillet 1982 (1982-07), pages 2333-41, XP002112749

## Description

La présente invention a pour objet l'hydrogénolyse d'halogène lourd porté par un carbone portant lui-même au moins un atome de fluor.

La présente invention a plus particulièrement pour objet un procédé d'obtention de composés portant un atome de fluor et un atome d'hydrogène sur un carbone d'hybridation sp³, ledit carbone portant lui-même une fonction électro-attractrice. La présente invention porte plus particulièrement sur un procédé en phase liquide.

Les dérivés fluorés de nature aliphatique, c'est-à-dire les dérivés fluorés dans lesquels le fluor se trouve porté, au moins en partie, par un carbone sp³, sont en général obtenus par un échange du fluor avec un autre atome d'halogène. Cet échange se fait en général en utilisant l'acide fluorhydrique ou bien des sels de l'acide fluorhydrique.

Toutefois, un des problèmes rencontrés est qu'il est souvent difficile de faire l'échange entre le fluor et un halogène de nombre atomique plus élevé lorsque l'halogène à échanger est porté par un carbone portant lui-même un atome d'hydrogène.

C'est pourquoi il est assez difficile d'obtenir des composés où un carbone aliphatique porte à la fois un hydrogène et au moins un fluor. Une des voies proposées consiste à déshydrohalogéner (c'est-à-dire à éliminer une molécule d'acide halohydrique pour donner un composé éthylénique, puis d'hydrogéner ce composé éthylénique). Cette voie n'est pas possible pour tous les composés car il faut nécessairement un hydrogène en position bêta pour réaliser l'élimination de l'hydrogène et de l'halogène que l'on désire éliminer.
Il a été proposé dans le brevet GB 1364 495 de synthétiser certains composés perfluorés monohydrogénés (Rf-H) à partir d'iodure correspondant (Rf-I), mais cet emploi de dérivé iodé est très coûteux et les conditions de pression décrites dans ce document sont très élevées pour des cinétiques qui ne semblent pas très élevés.
Dans la demande de brevet européen EP0726244, on décrit la réduction, d'une structure cyclopropanique acide très spécifique mais qui ne porte pas de groupe électro-attracteur en en sus d'un chlore et d'un fluor (en effet la fonction acide est certes un groupe électro attracteur mais elle n'est pas reliée directement au carbone porteur du fluor et de l'halogène).
On a également proposé des procédés en voie gazeuse (notamment EP0657 413 A), mais outre les inconvénients afférents à la voie gazeuse, il semble difficile d'obtenir une sélectivité élevée en même temps qu'un taux de conversion élevé.

C'est pourquoi un des buts de la présente invention est de fournir un procédé en phase liquide qui permette le remplacement d'un halogène lourd par un hydrogène et ce avec un atome de fluor porté par le même carbone que celui qui porte l'halogène à remplacer par un hydrogène.

Un autre but de la présente invention est de fournir un procédé du type précédent qui soit sélectif vis-à-vis du fluor.

Un autre but de la présente invention est de fournir un procédé du type précédent qui soit susceptible de donner de bons résultats avec un composé ne présentant pas d'hydrogène en bêta de l'halogène à faire partir.

Un autre but de la présente invention est de fournir un procédé du type précédent qui soit sélectif vis-à-vis du fluor sans nécessiter l'utilisation d'iodure.

Ces buts, et d'autres, qui apparaîtront par la suite sont atteints au moyen d'un procédé d'hydrogénodéshalogénation (c'est-à-dire l'opération qui consiste à éliminer l'halogène d'une molécule en traitant cette dernière au moyen d'hydrogène pour donner d'une part de l'acide halohydrique et d'autre part la molécule initiale modifiée par le remplacement d'un halogène par un hydrogène) sélective qui comporte une étape de mise en contact d'un substrat présentant un atome de carbone d'hybridation sp³ porteur :
- d'un groupe électroattracteur (GEA) chargé négativement, choisi parmi les fonctions carboxyliques, sulfoniques, sulfiniques,
- d'au moins un atome de fluor :

- et d'au moins un atome d`halogène plus lourd que le fluor et choisi parmi le chlore et le brome ;

avec un réactif comportant :
- une phase aqueuse,
- une base,
- un métal appartenant au groupe VIII et à la quatrième ou à la sixième période du tableau périodique,
- et de l'hydrogène dissous dans la phase aqueuse, à une concentration en équilibre avec une phase gazeuse dont la pression partielle en hydrogène est au moins égale à 50 kPa, avantageusement comprise entre 50 kPa et 2.10⁷ Pa.

La présente invention vise plus particulièrement le cas où ledit atome d'hybridation sp³ est porteur de deux atomes de fluor.

Les fonctions électro-attractices préférées sont celles dont la constante de Hammett σₚ est au moins égale à 0,1 et il est également préférable que la composante inductive de σₚ, σᵢ soit au mois égale à 0,2, avantageusement à 0,3 (par exemple, cf. March "advanced organic chemistry", 3^{ème} édition, John Wüey and son, pages 242 à 250 et notamment tableau 4).

Parmi les groupes électro-attracteurs (GEA), on peut citer :
- les fonctions carboxyliques, sulfoniques, sulfiniques, c'est-à-dire les fonctions qui dérivent des acides carboxyliques, sulfoniques et sulfiniques [Ces fonctions peuvent être la fonction acide proprement dite (sous forme salfiée) mais aussi les amide et les imides.]

D'une manière générale, le procédé marche particulièrement bien lorsque le groupe électro-attracteur (GEA) correspond à une fonction acide salifiée.

En d'autres termes, le groupe électro-attracteur (GEA) est choisi alors parmi les groupes chargés négativement.

Parmi les métaux du groupe VIII, on préfère le nickel et le cobalt et, plus particulièrement, le nickel.

Dans la présente demande, il est fait référence au tableau de la classification périodique des éléments publiés au supplément du bulletin de la Société chimique de France en janvier 1966).

En effet, les métaux de la mine du platine, présentent une sélectivité relativement médiocre vis-à-vis du fluor à éliminer. Toutefois la période du platine est préférable à celle du palladium.

Les formes les plus utilisables dans le procédé sont les formes de catalyseurs solides et, plus particulièrement pour le nickel et le cobalt, les formes dites "de Raney".

Les catalyseurs préférés sont les catalyseurs à base de nickel de Raney, c'est-à-dire les catalyseurs dont le principal élément actif, de préférence le seul élément actif, est le nickel de Raney.

Les substrats ne présentent en général pas plus de 50 atomes de carbone, et même pas plus de 25. Il convient toutefois de souligner que le procédé ne présente pas les mêmes limitations que les voies en phase gazeuse et donc que la masse moléculaire ne présente pas de caractère critique.

Pour obtenir un bon rendement et une bonne sélectivité, il est très souhaitable de mener la réaction en maintenant le pH à une valeur suffisante pour ioniser l'éventuelle fonction acide et, plus généralement, au moins égale à 4, avantageusement à 7, de préférence à 10.

La quantité de base à introduire dans le milieu réactionnel est au moins égale à la quantité nécessaire à la neutralisation de l'acide halohydrique dégagé au cours de l'hydrogénodéshalogénation sélective et, le cas échéant, la quantité de base nécessaire à la neutralisation des fonctions acides du substrat lorsque ce dernier présente de telles fonctions. Il est rare que la quantité de base excède trois, et même deux, fois la quantité nécessaire à la neutralisation de l'acide halohydrique dégagé et à la neutralisation des fonctions acides du substrat.

En général, l'halogène plus lourd que le fluor est le chlore. En effet, le chlore est l'halogène préféré non du point de vue technique mais du point de vue économique. Le choix du chlore rend plus difficile la sélectivité de l'hydrogénodéshalogénation vis-à-vis du fluor. La présente invention présente peu d'intérêt pour les cas où l'halogène est l'iode, en effet la sélectivité entre le fluor et l'iode est telle que l'effet du présent procédé est moins marqué que dans le cas du brome et a fortiori du chlore.

La réaction de hydrogénodéshalogénation est avantageusement menée à une température comprise entre la température ambiante (environ 20°C) et environ 150°C. Dans la présente description le terme "environ" est employé pour mettre en exergue le fait que les valeurs qui le suivent correspondent à des arrondis mathématiques et notamment que lorsque le ou les chiffres les plus à droite d'un nombre sont des zéros, ces zéros sont des zéros de position et non des chiffres significatifs, sauf bien entendu s'il en est précisé autrement.
En général, on préfère mener l'hydrogénodéshalogénation à une température comprise entre 30°C et 100°C (deux chiffres significatifs).

Les bases n'ont pas besoin d'être complètement solubles dans le milieu réactionnel. Il suffit qu'elles le soient un peu et qu'elles maintiennent le pH aux valeurs désirées.

On peut notamment citer les oxydes et les sels basiques d'alcalins, d'alcalino-terreux, ainsi que les hydroxydes correspondants.

Les bases organiques peuvent aussi être utilisées soit seules, soit pour faciliter le transfert des ions OH⁻/H⁺. Parmi ces dernières, on peut citer les hydroxydes d'ammonium, les amines primaires, secondaires ou tertiaires. On peut également utiliser d'autres agents de transfert de phase, notamment des "cryptants" tels que des éthers couronnes.

Lorsque les bases organiques sont utilisées pour faciliter l'action d'une base peu soluble (notamment minérale), on peut les utiliser à hauteur d'environ 0,1 fois la quantité de substrat exprimée en moles (ou plus généralement en équivalent). Dans le cas de l'utilisation conjointe d'une base minérale et d'une amine il est inutile que la quantité d'amine soit supérieure à 0,4 fois la quantité de substrat exprimée en équivalent. Les amines préférées sont celles qui ne sont pas facilement alcoylables et notamment les tertiaires.

Il peut être intéressant de prévoir un tiers solvant pour aider le substrat à être au moins partiellement soluble dans la phase aqueuse.

Il est préférable de choisir comme tiers solvant un des solvants miscibles, partiellement ou de préférence en toute proportion, avec l'eau, mais moins polaire que cette dernière.

Il est préférable que ces solvants ne soient pas susceptibles d'être hydrogénés dans les conditions de la réaction. Cette contrainte peut conduire à exclure en particulier les cétones et les nitriles ou à choisir des conditions douces.

Aussi, parmi les solvants qui peuvent être envisagés, il convient de citer les éthers, les alcools et leurs mélanges.

D'une manière plus générale, il est souhaitable que les constituants du mélange réactionnel et surtout le substrat ne comporte pas de fonction susceptible d'être hydrogénée dans les conditions de la réaction.

Les substrats répondent en général à la formule générale suivante :

GEA-CFX-Y

où X représente un halogène de rang supérieur à celui du fluor (c'est-à-diré essentiellement chlore et brome, de préférence chlore) ;
où Y représente un hydrogène (mais cela n'est pas la valeur préférée), un atome d'halogène (avantageusement un fluor), un radical carboné avantageusement électro-attracteur, ou même un groupe électro-attracteur (tel que défini dans la présente description et notamment ci dessous) ;
où GEA représente un groupe électro-attracteur chargé négativement dont les éventuelles fonctions sont inertes dans les conditions de la réaction, avantageusement choisi parmi -C(O)-O- ; -S(O)-O- ; -S(O)₂-O.

Le nombre total de carbone du substrat étant avantageusement compris dans l'intervalle fermé 1 à 15, de préférence 2 à 10 (hors la partie carbonée des fonctions amide ou imide lorsque les substrats sont des acides sous l'une des formes précédentes).
Y est avantageusement :
- fluor
- si l'on désire obtenir un méthyle monofluoré (-CH₂F), Y peut avoir les mêmes valeurs que X avec les mêmes sous-préférences
- un reste de formule (II) R-(CΞ₂)ₚ-
   où les Ξ, semblables ou différents, représentent un fluor ou un radical perfluoré de formule CₙF₂ₙ₊₁, avec n entier au plus égal à 8, avantageusement à 5 ;
   où p représente un entier au plus égal à 2;
   où R est un atome d'hydrogène, un atome de fluor ou un radical hydrocarboné, avantageusement alcoyle ou aryle.

Parmi les substrats préférés, on peut citer plus particulièrement les acides carboxyliques mono- ou bi-fluorés sur l'atome de carbone porteur de la fonction carboxylique et, en particulier, ceux dont le carbone en alpha est à la fois chloré et fluoré.

Le substrat peut, mais cela ne présente que rarement un intérêt, comporter plusieurs sites de type GEA-CFX-, auquel cas les différents X seront remplacés simultanément par l'hydrogène

Le procédé s'est révélé particulièrement intéressant pour les acides carboxyliques fluorés dont le carbone en alpha est chloré, en particulier l'acide chlorodifluoroacétique qui permet d'obtenir l'acide difluoroacétique.

Ces fonctions carboxyliques dont on a parlé précédemment sont avantageusement utilisées sous la forme de sels, en général alcalins, d'acide.

Ils peuvent toutefois être utilisés sous d'autres formes, notamment les fonctions dérivées des acides qui ont été mentionnées ci-dessus (par exemple, imide ou amide).

La stoechiométrie de la réaction est :

GEA-CFX-Y + H2 → HX + GEA-CFH-Y

et lorsque Y est choisi parmi les valeurs de X :

GEA-CFX-Y + 2 H₂ → HX + HY + GEA-CFH₂

Les exemples non limitatifs suivants illustrent l'invention :

### Exemple 1

Dans un réacteur SOTELEM de 300 ml en Hastelloy HB2, on introduit 57 g d'eau, on agite et, en refroidissant, on ajoute 57 g d'acide chlorodifluoroacétique (0,44 mole). Toujours en refroidissant, on introduit 137 g d'une solution aqueuse 10 N d'hydroxyde de sodium (environ 1,2 mole). On ajoute ensuite 1 g de nickel de Raney. On ferme le réacteur, on purge à l'azote par 2 fois 10 bar et à l'hydrogène par 2 fois 10 bar.

On place le réacteur sous 20 bar de pression et on chauffe à 70°C en agitant. On maintient le réacteur sous une pression constante de 20 bar. Lorsque la consommation d'hydrogène cesse, on maintient dans ces conditions encore 15 mn, puis on refroidit à 20°C ; la consommation d'hydrogène est alors sensiblement égale à la quantité stoechiométrique. On purge à l'azote par 2 fois 10 bar.

Le catalyseur est filtré. Par analyse du milieu réactionnel par chromatographie ionique, on obtient un TT = 99,8% avec un RR difluoroacétate de sodium de 98,7%.

### Exemple 2 comparatif

On procède comme dans l'exemple 1, mais en utilisant comme catalyseur 0,50 g de Pd/C à 5% de palladium. Les résultats sont les suivants : Consommation d'hydrogène = environ 50% de la QS (c'est-à-dire Quantité Stoechiométrique) :
- TT = 25%
- ^{RR}difluoroacétate de sodium = 12%
- RT= 50%
- ^{RR}acétate de sodium = 10%

### Exemple 3 comparatif

On procède comme dans l'exemple 1, mais en supprimant la soude. Les résultats sont les suivants : Consommation d'hydrogène = environ 10% de la QS (c'est-à-dire Quantité Stoechiométrique) :
- TT <10%
- Présence d'ion fluor en quantité significative
- Présence d'acide acétique
- Présence de fluorure de nickel
- Présence de DFA (difluoroacétate difluoroacétique) en très faible quantité.

## Revendications

1. Procédé d'hydrogénodéshalogénation sélective, **caractérisé par le fait qu'**il comprend l'étape de mise en contact d'un substrat présentant un atome de carbone d'hybridation sp³ porteur
- d'un groupe électroattracteur chargé négativement, choisi parmi les fonctions carboxyliques, sulfoniques, sulfiniques,
- d'au moins un atome de fluor ;
- et d'au moins un atome d'halogène plus lourd que le fluor et choisi parmi le chlore et le brome ;
avec un réactif comportant :
. une phase aqueuse,
. une base,
. un métal appartenant au groupe VIII et à la quatrième ou à la sixième période du tableau périodique,
. et de l'hydrogène dissous dans la phase aqueuse, à une concentration en équilibre avec une phase gazeuse dont la pression partielle en hydrogène est au moins égale à 50 kPa, avantageusement comprise entre 50 kPa et 2.10⁷ Pa.

2. Procédé selon la revendication 1, **caractérise par le fait que** ledit atome de carbone d'hybridation sp³ est porteur de deux atomes de fluor.

3. Procédé selon l'une des revendications 1 et 2, **caractérisé par le fait que** ledit métal du groupe VIII est choisi parmi le nickel et le cobalt.

4. Procédé selon l'une des revendications 1 à 3, **caractérisé par le fait que** ledit métal du groupe VIII est choisi parmi le nickel ou le cobalt est sous la forme dite de Raney.

5. Procédé selon l'une des revendications 1 à 4**, caractérisé par le fait que** ledit métal du groupe VIII est le nickel de Raney.

6. Procédé selon l'une des revendications 1 à 5, **caractérisé par le fait que** le pH (mesuré dans les conditions normales) de ladite phase aqueuse est maintenu à une valeur au moins égale à 4, avantageusement à 7, de préférence à 10.

7. Procédé selon l'une des revendications 1 à 6, **caractérisé par le fait que** le substrat est un acide et que la quantité de base introduite est au moins égale à la quantité nécessaire à la neutralisation dudit acide et à celle de l'acide halohydrique dégagé par l'hydrogénodéshalogénation sélective.

8. Procédé selon l'une des revendications 1 à 7, **caractérisé par le fait que** ledit halogène plus lourd que le fluor est le chlore.

9. Procédé selon l'une des revendications 1 à 8, **caractérisé par le fait que** ladite étape est menée à une température comprise entre l'ambiante et 150°C.

10. Procédé selon l'une des revendications 1 à 9, **caractérisé par le fait que** ladite étape est menée à une température comprise entre 30°C et 100°C.

11. Procédé selon l'une des revendications 1 à 10, **caractérisé par le fait que** ladite base est choisie parmi les hydroxydes, les carbonates et les sels basiques, d'alcalins, d'alcalino-terreux, ammonium et leurs mélanges.

12. Procédé selon l'une des revendications 1 à 11, **caractérisé par le fait que** la phase aqueuse comporte un tiers solvant pour aider à solubiliser le substrat, ledit tiers solvant étant choisi parmi les solvants miscibles avantageusement en toute proportion avec l'eau, mais moins polaire que cette dernière.

13. Procédé selon l'une des revendications 1 à 12 **caractérisé par le fait que** le solvant est choisi parmi les solvants, les éthers, les alcools et leurs mélanges.

14. Procédé selon l'une des revendications 1 à 13, **caractérisé par le fait que** ledit substrat est choisi parmi les acides carboxyliques fluorés, dont le carbone en alpha est chloré, les aralcoyles dont le carbone en position benzylique est fluoré et chloré, les éthers dont au moins un des carbones porteurs de la fonction éther est à la fois chloré et fluoré.

15. Procédé selon l'une des revendications 1 à 14, **caractérisé par le fait que** ledit substrat est choisi parmi les acides carboxyliques fluorés, dont le carbone en alpha est chloré, en particulier l'acide chlorodifluoroacétique.

## Claims

1. Selective hydrodehalogenation process, **characterized in that** it comprises the stage in which a substrate exhibiting a carbon atom of sp³ hybridization carrying:
- a negatively charged electron-withdrawing group chosen from carboxylic, sulphonic and sulphinic functional groups;
- at least one fluorine atom;
- and at least one halogen atom heavier than fluorine and chosen from chlorine and bromine;
is brought into contact with a reactant comprising:
• an aqueous phase,
• a base,
• a metal belonging to Group VIII and to the fourth or to the sixth period of the Periodic Table
• and hydrogen dissolved in the aqueous phase, at a concentration in equilibrium with a gas phase, the hydrogen partial pressure of which is at least equal to 50 kPa, advantageously between 50 kPa and 2 × 10⁷ Pa.

2. Process according to Claim 1, **characterized in that** the the said carbon atom of sp³ hybridization carries two fluorine atoms.

3. Process according to either of Claims 1 and 2, **characterized in that** the said metal from Group VIII is chosen from nickel and cobalt.

4. Process according to one of Claims 1 to 3, **characterized in that** the said metal from Group VIII is chosen from nickel or cobalt in the "Raney" form.

5. Process according to one of Claims 1 to 4, **characterized in that** the said metal from Group VIII is Raney nickel.

6. Process according to one of Claims 1 to 5, **characterized in that** the pH (measured under standard conditions) of the said aqueous phase is maintained at a value at least equal to 4, advantageously to 7, preferably to 10.

7. Process according to one of Claims 1 to 6, **characterized in that** the substrate is an acid and **in that** the amount of base introduced is at least equal to the amount necessary for the neutralization of the said acid and for that of the hydrohalic acid given off by the selective hydrodehalogenation.

8. Process according to one of Claims 1 to 7, **characterized in that** the said halogen heavier than fluorine is chlorine.

9. Process according to one of Claims 1 to 8, **characterized in that** the said stage is carried out at a temperature of between ambient temperature and 150°C.

10. Process according to one of Claims 1 to 9, **characterized in that** the said stage is carried out at a temperature of between 30°C and 100°C.

11. Process according to one of Claims 1 to 10, **characterized in that** the said base is chosen from alkali metal, alkaline earth metal and ammonium hydroxides, carbonates and basic salts and their mixtures.

12. Process according to one of Claims 1 to 11, **characterized in that** the aqueous phase comprises a third solvent to help in dissolving the substrate, the said third solvent being chosen from solvents which are advantageously miscible in any proportion with water but which are less polar than the latter.

13. Process according to one of Claims 1 to 12, **characterized in that** the solvent is chosen from ethers, alcohols and their mixtures.

14. Process according to one of Claims 1 to 13, **characterized in that** the said substrate is chosen from fluorinated carboxylic acids in which the α carbon is chlorinated, aralkyls in which the carbon in the benzyl position is fluorinated and chlorinated, and ethers, at least one of the carbons of which which carry the ether functional group is both chlorinated and fluorinated.

15. Process according to one of Claims 1 to 14, **characterized in that** the said substrate is chosen from fluorinated carboxylic acids in which the α carbon is chlorinated, in particular chlorodifluoroacetic acid.

## Patentansprüche

1. Verfahren zur selektiven Hydrodehalogenierung, **dadurch gekennzeichnet, dass** es den Schritt des Inkontaktbringens eines Substrats, das ein sp³-hybridisiertes Kohlenstoffatom aufweist, das:
- eine negativ geladene elektronenziehende Gruppe, die unter den Carboxyl-, Sulfon-, Sulfinfunktionen ausgewählt ist;
- wenigstens ein Fluoratom;
- und wenigstens ein Halogenatom, das schwerer als Fluor ist und unter Chlor und Brom ausgewählt ist;
trägt, mit einem Reaktanten umfasst, der
- eine wässrige Phase,
- eine Base,
- ein Metall, das der Gruppe VIII und der vierten oder sechsten Periode des Periodensystems angehört,
- und Wasserstoff umfasst, der in der wässrigen Phase mit einer Konzentration gelöst ist, die im Gleichgewicht mit einer Gasphase steht, deren Wasserstoff partialdruck wenigstens gleich 50 kPa ist, vorteilhafterweise zwischen 50 kPa und 2*-*10⁷Pa liegt.

2. Verfahren gemäß Anspruch 1, **dadurch gekennzeichnet, dass** besagtes sp³-hybridisiertes Kohlenstoffatom zwei Fluoratome trägt

3. Verfahren gemäß einem der Ansprüche 1 und 2, **dadurch gekennzeichnet, dass** besagtes Metall der Gruppe VIII unter Nickel und Kobalt ausgewählt ist.

4. Verfahren gemäß einem der Ansprüche 1 bis 3, **dadurch gekennzeichnet, dass** besagtes, unter Nickel oder Kobalt ausgewähltes Metall der Gruppe VIII in der sogenannten Raney-Form vorliegt

5. Verfahren gemäß einem der Ansprüche 1 bis 4, **dadurch gekennzeichnet, dass** besagtes Metall der Gruppe VIII Raney-Nickel ist

6. Verfahren gemäß einem der Ansprüche 1 bis 5, **dadurch gekennzeichnet, dass** der pH (gemessen unter Normalbedingungen) besagter wässriger Phase auf einem Wert von wenigstens gleich 4, vorteilhafterweise 7, vorzugsweise 10, gehalten wird.

7. Verfahren gemäß einem der Ansprüche 1 bis 6, **dadurch gekennzeichnet, dass** das Substrat eine Säure ist und dass die Menge an zugesetzter Base wenigstens gleich der Menge, die für die Neutralisation besagter Säure notwendig ist, und der Menge an Halogerrwasserstoffsäure, die durch die selektive Hydrodehalogenierung freigesetzt wird, ist.

8. Verfahren gemäß einem der Ansprüche 1 bis 7, **dadurch gekennzeichnet, dass** besagtes Halogen, das schwerer als Fluor ist, Chlor ist.

9. Verfahren gemäß einem der Ansprüche 1 bis 8, **dadurch gekennzeichnet, dass** besagter Schritt bei einer Temperatur ausgeführt wird, die zwischen Raumtemperatur und 150°C liegt.

10. Verfahren gemäß einem der Ansprüche 1 bis 9, **dadurch gekennzeichnet, dass** besagter Schritt bei einer Temperatur ausgeführt wird, die zwischen 30 °C und 100 °C liegt.

11. Verfahren gemäß einem der Ansprüche 1 bis 10, **dadurch gekennzeichnet, dass** besagte Base unter den Hydroxiden, den Carbonaten und den basischen Salzen von Alkalimetallen, Erdalkalimetallen, Ammonium und ihren Gemischen ausgewählt ist.

12. Verfahren gemäß einem der Ansprüche 1 bis 11, **dadurch gekennzeichnet, dass** die wässrige Phase ein drittes Lösungsmittel umfasst, um das Lösen des Substrats zu unterstützen, wobei besagtes drittes Lösungsmittel unter den Lösungsmitteln ausgewählt ist, die vorteilhafterweise in jedem Verhältnis mit Wasser mischbar, aber weniger polar als dieses Letztere sind.

13. Verfahren gemäß einem der Ansprüche 1 bis 12, **dadurch gekennzeichnet, dass** das Lösungsmittel unter den Lösungsmitteln, den Ethern, den Alkoholen und ihren Gemischen ausgewählt ist.

14. Verfahren gemäß einem der Ansprüche 1 bis 13, **dadurch gekennzeichnet, dass** besagtes Substrat ausgewählt ist unter den fluorierten Carbonsäuren, deren alpha-Kohlenstoff chloriert ist, den Aralkylen, deren Kohlenstoff in Benzylposition fluoriert und chloriert ist, den Ethem, bei denen wenigstens einer der Kohlenstoffe, die die Etherfunktion tragen, gleichzeitig chloriert und fluoriert ist.

15. Verfahren gemäß einem der Ansprüche 1 bis 14, **dadurch gekennzeichnet, dass** besagtes Substrat ausgewählt ist unter den fluorierten Carbonsäuren, deren alpha-Kohlenstoff chloriert ist insbesondere Chlordifluvressigsäure.
